# EUROPEAN PATENT APPLICATION

(11) **EP 1 507 001 A1**
(43) Date of publication of application: **16.02.2005**
(21) Application number: 03730557.0
(22) Date of filing: 22.05.2003
(51) Int. Cl.: C12N 9/04, C12P 7/58

(54) **NOVEL (D)-2-HYDROXY ACID OXIDASE DERIVED FROM MICROORGANISM AND BIOCHEMICAL PROCESS FOR PRODUCING GLYOXYLIC ACID THEREWITH**

(30) Priority: 24.05.2002 JP 2002150850
(71) Applicant: Kaneka Corporation, Osaka 530-8288 (JP)
(72) Inventor: IWASAKI, Akira, Kakogawa-shi, Hyogo 675-0068 (JP); MATSUMOTO, Takehiko, Takasago-shi, Hyogo 676-0807 (JP); WASHIDA, Motohisa, Tarumi-ku, Kobe-shi, Hyogo 655-0872 (JP); WATANABE, Hiroshi, Nishi-ku, Kobe-shi, Hyogo 651-2276 (JP); SHIMIZU, Sakayu, Ukyo-ku, Kyoto-shi, Kyoto 616-8212 (JP)
(74) Representative: HOFFMANN - EITLE
(86) International application number: PCT/JP2003/006367
(87) International publication number: WO 2003/100043

(57) **Abstract**

The present invention provides an industrially advantageous process for producing glyoxylic acid by a biochemical procedure using oxidase. In particular, the present invention provides novel (D)-2-hydroxy-acid oxidase acting on a 2-hycoxy acid to produce a corresponding 2-keto acid, and a process for producing glyoxylic acid in which the 2-hydroxy-acid oxidase, a culture solution of microorganisms capable of producing the enzyme, microorganism cells isolated from the culture solution, or a product of treatment of microorganism cells acts on glycolic acid to convert the glycolic acid to glyoxylic acid.

## Description

### Technical Field

The present invention relates to novel (D)-2-hydroxy-acid oxidase derived from microorganisms having an ability to convert glycolic acid to glyoxylic acid. The present invention also relates to a biochemical process for producing glyoxylic acid comprising converting glycolic acid to glyoxylic acid by oxidization with the oxidase and/or microorganisms capable of producing the oxidase or a treatment product thereof.

### Background Art

Glyoxylic acid is used as a raw material for synthesizing vanillin, ethyl vanillin, and the like, and is also a useful compound as an intermediate for synthesizing agricultural chemicals and pharmaceutical products. A conventional known process for producing glyoxylic acid is a chemical process such as oxidation of glyoxal with nitric acid. At present, glyoxylic acid is mostly produced by the chemical process. However, the chemical process such as oxidation of glyoxal with nitric acid easily produces by-products such as an organic acid other than glyoxylic acid and causes an undesirable effect on the quality of the produced glyoxylic acid. Therefore, a complicated step is required for removing the by-products. The chemical process is also disadvantageous in that a large amount of salt waste produced in a step of neutralizing nitric acid or the like used in large amounts must be treated.

On the other hand, a known biochemical process for producing glyoxylic acid is a process of converting glycolic acid to glyoxylic acid using glycolate oxidase derived from spinach (PCT Japanese Translation Patent Publication Nos. 7-502895 and 8-508159). The glycolate oxidase is a well-known enzyme which is commonly found in green plants. However, the glycolate oxidase derived from plants such as spinach has relatively high activity for glyoxylic acid. Consequently, when the glycolate oxidase is used, glyoxylic acid is further oxidized to produce by-products such as oxalic acid. Therefore, a large amount of an amine must be added for preventing the production of by-products such as oxalic acid, and the use of a large amount of an expensive amine causes a great industrial problem. Furthermore, the process using glycolate oxidase derived from plants uses an expensive enzyme produced by extraction from plants and thus has the problem of high-cost industrial production.

On the other hand, Isobe et al. report that glycerol oxidase produced from Aspergillus japonica has an activity to oxidize glycolic acid. However, the activity of glycerol oxidase for glycolic acid is low, and thus a large amount of the enzyme is required for accumulating glyoxylic acid (Japanese Unexamined Patent Application Publication No. 7-163380 and Biosci. Biotech. Biochem., 59(4), 576-581, 1995). Isobe et al. also report that microorganisms of the genera Geotrichum, Gluconobacter, and Acetobacter, which are glycerol dehydrogenase-producing microorganisms, other than glycerol oxidase derived from the genus Aspergillus have the ability of converting glycolic acid to glyoxylic acid and accumulating glyoxylic acid. Therefore, Isobe et al. suggest the conversion of glycolic acid to glyoxylic acid using glycerol dehydrogenase (Japanese Unexamined Patent Application Publication No. 7-163380). It is also reported that microorganisms of the genus Pseudomonas or Alcaligenes have the ability of converting glycolic acid to glyoxylic acid and accumulating glyoxylic acid. However, detailed knowledge about an enzyme for converting glycolic acid to glyoxylic acid using these strains is not reported (Japanese Unexamined Patent Application Publication No. 8-322581). Although there have been some reports on the conversion of glycolic acid to glyoxylic acid using glycerol oxidase or glycerol dehydrogenase derived from microorganisms or the microorganisms, the enzyme or the microorganisms are not at a level suitable for industrial application in view of activity and percentage of completion.

When oxidase is used in the oxidation reaction of glycolic acid to produce glyoxylic acid, only oxygen other than glycolic acid used as a substrate is generally required. Unlike in the use of dehydrogenase, it is unnecessary to add an oxidized coenzyme such as NAD or NADP, and an enzyme and substrate for converting a reduced coenzyme produced by reaction to the oxidized form. Therefore, the use of oxidase in the oxidation reaction of glycolic acid to produce glyoxylic acid is an industrially advantageous method. As described above, however, an oxidase-type enzyme exhibiting high activity and selectivity for glycolic acid has not been found so far.

### Disclosure of Invention

Accordingly, an object of the present invention is to provide oxidase derived from microorganisms having an activity to convert glycolic acid to glyoxylic acid, and a process for effectively producing glyoxylic acid using the oxidase derived from microorganisms or microorganisms capable of producing the enzyme.

As a result of intensive research for developing a process for effectively producing glyoxylic acid, the inventors found microorganisms having high oxidase activity for glycolic acid from soil, and isolated and purified an enzyme having the activity from the microorganisms. The detailed research of the enzyme resulted in the completion of the present invention. Namely, the present invention relates to a novel oxidase for converting glycolic acid to glyoxylic acid, and relates to a process for producing glyoxylic acid comprising treating glycolic acid with microorganism cells capable of producing the enzyme and/or a treatment product of the microorganism cells to convert glycolic acid to glyoxylic acid, accumulating glyoxylic acid, and collecting it.

### Brief Description of the Drawings

Fig. 1 is a diagram showing the principle of a method for measuring the reaction activity of oxidase.
Fig. 2 is a graph showing the temperature stability of an enzyme of the present invention.
Fig. 3 is a graph showing the optimum pH for reaction of an enzyme of the present invention, in which a solid square shows the use of a 0.1 M phosphate buffer, and a blank square shows the use of a 0.1 M Tris-HCl buffer.
Fig. 4 is a graph showing the pH stability of an enzyme of the present invention.

### Best Mode for Carrying Out the Invention

An enzyme of the present invention for converting glycolic acid to glyoxylic acid is (D)-2-hydroxy-acid oxidase mainly characterized by the following physicochemical properties (1) and (2):
(1) action: acting on 2-hydroxy acid to produce a corresponding 2-keto acid; and
(2) substrate specificity: exhibiting activity for glycolic acid and D-lactic acid, but no activity for L-lactic acid.
   The enzyme of the present invention, (D)-2-hydroxy-acid oxidase, may also have the following physicochemical property (3):
(3) exhibiting no activity for L-2-hydroxyisocaproic acid, methanol, ethanol, 1-propanol, 2-propanol, ethylene glycol, 1,2-propanediol, and glycerin.
   The enzyme of the present invention, (D)-2-hydroxy-acid oxidase, may further have the following physicochemical properties:
(4) molecular weight: about 60,000 Da in SDS polyacrylamide gel electrophoresis analysis;
(5) stability: maintaining 90% or more of activity after treatment at pH 7.2 and 40°C for 20 minutes; and
(6) optimum reaction pH: 7 to 10.

The microorganisms used as an origin of the (D)-2-hydroxy-acid oxidase of the present invention are not particularly limited. However, for example, bacteria of the genus Arthrobacter are used. A preferred origin is Arthrobacter sp. KNK-GA1 strain (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375) which was isolated from soil and identified by the inventors for the first time. The mycological properties of the Arthrobacter sp. KNK-GA1 strain (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375) (hereinafter simply referred to as the "KNK-GA1 strain" (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375)) are given below.

| | |
|---|---|
| Cell shape | rod shape |
| Gram's staining | + |
| Sporogenesis | - |
| Motility | - |
| Colony shape | cream-colored circular shape with gloss, a smooth periphery, and low convexity |
| Growth (37°C) | + |
| (41°C) | - |
| Catalase | + |
| Oxidase | - |
| OF test (glucose) | - |

The (D)-2-hydroxy-acid oxidase derived from the KNK-GA1 strain (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375) can be isolated and purified, for example, as described below.

First, the KNK-GA1 strain (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375) is cultured in an appropriate medium. The medium used for the culture is not particularly limited. A preferred example of the medium is a medium (pH 7) having a composition containing 5 g/L of glycolic acid, 1 g/L of a yeast extract, 3.5 g/L of potassium dihydrogen phosphate, 6.5 g/L of diammonium hydrogen phosphate, 0.5 g/L of magnesium sulfate heptahydrate, 0.02 g/L of zinc sulfate heptahydrate, 0.03 g/L of ferrous sulfate heptahydrate, 0.002 g/L of copper sulfate pentahydrate, 0.1 g/L of calcium chloride dihydrate, and 0.3 g/L of sodium chloride.

Then, the cells are collected from the culture solution by centrifugation, and suspended in a 0.05 M phosphate buffer (pH 7). The resultant suspension is crushed with Dynomill (produced by Dyno-Mill Co., Ltd.), and the remaining cells are removed by centrifugation to prepare a supernatant (cell-free extract). Then, protamine sulfate is added to the supernatant, and the produced insoluble substance is removed by centrifugation to remove nucleic acids.

The target enzyme having activity is further purified from the protamine-treated solution by salting out (ammonium sulfate or the like) or any of various types of chromatography such as ion exchange chromatography, hydrophobic chromatography, and adsorption chromatography.

The (D)-2-hydroxy-acid oxidase of the present invention may be a natural enzyme or recombinant enzyme as long as the enzyme has substantially the same properties as those of (D)-2-hydroxy-acid oxidase derived from the KNK-GA1 strain (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375). For example, the recombinant enzyme can be produced by replacing or deleting at least one of the amino acids in the amino acid sequence of the (D)-2-hydroxy-acid oxidase derived from the KNK-GA1 strain (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375), or inserting or adding at least one amino acid. The (D)-2-hydroxy-acid oxidase may be produced by mutating the KNK-GA1 strain (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375), or produced from other natural or non-natural microorganisms.

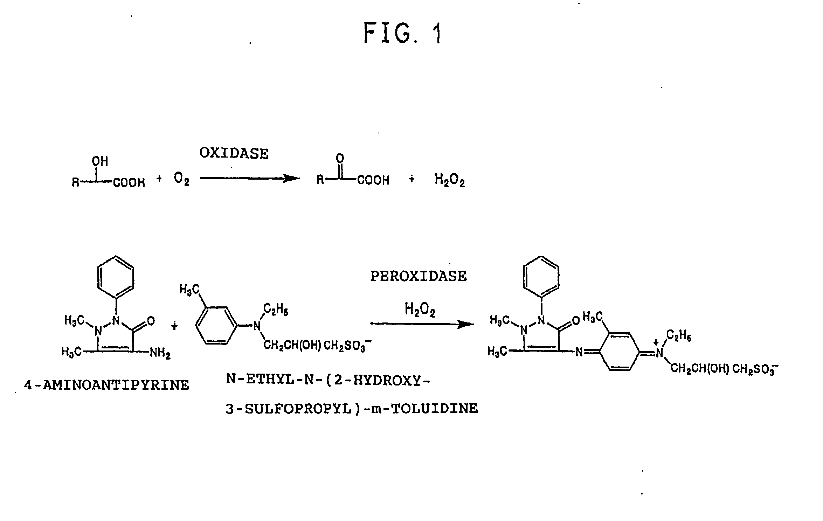

As shown in Fig. 1, in the present invention, the reaction activity of oxidase is measured by reacting H₂O₂, which is produced by oxidation reaction, with 4-aminoantipyrine and N-ethyl-(2-hydroxy-3-sulfopropyl)-m-toluidine in the presence of peroxidase, and then measuring absorption of the produced quinine imine dye.

The reaction activity of oxidase is basically measured by adding 0.1 ml of an enzyme solution to 0.9 ml of a 100 mM phosphate buffer (pH 7) having the composition below, and measuring an increase in absorbance at a wavelength of 555 nm at 30°C. In the present invention, when 1 µmol of H₂O₂ is produced for 1 minute, the enzyme activity is defined as 1 unit.

### Composition:

| | |
|---|---|
| Glycolic acid | 10 mM |
| 4-aminoantipyrine (referred to as "4-AA" hereinafter) | 0.67 mM |
| N-ethyl-N-(2-hydroxy-3-sulfopropyl)-m-toluidine (referred to as "TOOS" hereinafter) | 1.09 mM |
| Peroxidase (referred to as "POD" hereinafter) derived from horseradish | 2 U/ml |

The amounts of glycolic acid and glyoxylic acid can be determined by high-performance liquid chromatographic analysis. The high-performance liquid chromatographic analysis is performed by using, for example, a BioRad Aminex HPX-87H (7.8 mm × 300 mm) column and a 5 mM H₂SO₄ aqueous solution as a solvent at a flow rate of 0.4 ml/min. Detection is performed by measuring absorbance at 230 mM. Under these conditions, glycolic acid and glyoxylic acid are eluted 20 minutes and 15 minutes, respectively, after the start.

Table 1 shows the substrate specificities of the (D)-2-hydroxy-acid oxidase of the present invention and glycolate oxidases derived from plants and algae, such as spinach, Lemna minor (J. Biochem., 16, 1373-1378, 1984), and Spatoglossum pacificum (J. Phycol., 32, 790-798, 1996). In Table 1, the activity of each enzyme for glycolic acid is regarded as 100%.

**Table 1**

| Comparison of substrate specificity between known glycolate oxidases derived from plants and oxygen of the present invention | | | | |
|---|---|---|---|---|
| | Relative Activity (%) | | | |
| | Enzyme of this invention | Spinach | Lemna minor | Spatoglossum pacificum |
| Glycolic acid | 100 | 100 | 100 | 100 |
| L-lactic acid | 0 | 81 | 60 | 26 |
| D-lactic acid | 82 | 0 | 7 | 0 |
| DL-2-hydroxybutyric acid | 42 | 71 | 57 | 55 |
| DL-2-hydroxyvaleric acid | 32 | 70 | 52 | 91 |
| DL-2-hydroxycaproic acid | 7 | 29 | 13 | ― |
| DL-2-hydroxyisocaproic acid | 10 | 51 | ― | ― |
| L-2-hydroxyisocaproic acid | 0 | 68 | 30 | 77 |
| Glyoxylic acid | 4 | 8 | 35 | 14 |

The known glycolate oxidases show activity for 2-hydroxy acid other than glycolic acid, and all the known glycolate oxidases show activity for L-lactic acid and L-2-hydroxyisocaproic acid, but show substantially no activity for D-lactic acid. At present, the known oxidases are classified as (S)-2-hydroxy-acid oxidases (EC 1.1.3.15) in view of stereospecificity. On the other hand, the (D)-2-hydroxy-acid oxidase of the present invention shows high activity for glycolic acid and D-2-hydroxy acids such as D-lactic acid, but shows no activity for L-lactic acid. Also, the (D)-2-hydroxy-acid oxidase of the present invention shows activity for DL-2-hydroxyisocaproic acid but no activity for L-2-hydroxyisocaproic acid. It is thus understood that the (D)-2-hydroxy-acid oxidase of the present invention has specific activity for the D-form. In addition, lactic acid oxidase derived from soil gram-negative bacteria has been reported as an enzyme acting on D-2-hydroxy acids (Appl. Biochem. Biotechnol., 56, 277-288, 1996). However, this enzyme has activity for L-lactic acid at a rate of 57% of the activity for D-lactic acid, and thus it has no reaction specificity for the D-form.

As described above, the enzyme of the present invention is characterized in that it is novel (D)-2-hydroxy-acid oxidase exhibiting activity for D-2-hydroxy acids and discovered from microorganisms exhibiting high activity for glycolic acid. The (D)-2-hydroxy-acid oxidase may be expressed by (R)-2-hydroxy-acid oxidase in another nomenclature system. The (D)-2-hydroxy-acid oxidase of the present invention is the first enzyme found to selectively act on D-2-hydroxy acids but not act on L-2-hydroxy acids. Although conventional glycolate oxidases show no or low reaction activity for a D-substrate, the (D)-2-hydroxy-acid oxidase of the present invention permits effective reaction of D-2-hydorxy acids for the first time. Therefore, the (D)-2-hydroxy-acid oxidase of the present invention is excellent not only in that glycolic acid is converted to glyoxylic acid, but also in that the (D)-2-hydroxy-acid oxidase can be used in reaction using a D-2-hydroxy acid as the substrate or optical resolution for selective reaction of the D-isomer.

As shown in Table 1, it is known that conventional glycolate oxidases ((S)-2-hydroxy-acid oxidase) show relatively high activity for glyoxylic acid, but the enzyme of the present invention shows relatively low activity for glyoxylic acid. Therefore, the enzyme of the present invention is advantageous in that only small amounts of by-products such as oxalic acid are produced by enzymatic oxidation of glyoxylic acid in the conversion of glycolic acid to glyoxylic acid

A process for producing glyoxylic acid of the present invention is mainly characterized in that glycolic acid is treated with any one of the (D)-2-hydroxy-acid oxidase derived from microorganisms, a culture solution of the enzyme-producing microorganisms, microorganism cells isolated from the culture solution, and a treatment product of the microorganism cells to convert glycolic acid to glyoxylic acid, and the glyoxylic acid is accumulated. Examples of the treatment product of the microorganism cells include a crude enzyme solution, lyophilized cells, acetone-dried cells, and crushed products thereof. Examples of the crude enzyme solution include a solution prepared by centrifugally separating cells from a culture solution, suspending the cells in an appropriate buffer, and crushing or dissolving the cells by a physical means using glass beads or a biochemical means using an enzyme; and a cell-free extract prepared by centrifugally removing a solid from the solution. The cell-free extract may be partially purified by a method conventionally used by persons skilled in the art, for example, dialysis, ammonium sulfate precipitation, or chromatography, to produce an enzyme solution which may be used as the crude enzyme solution. These conventional purification methods may be used alone or in combination. The treatment product of the microorganism cells may be immobilized in the form of an enzyme or bacteria by a known means. The immobilization can be performed by a method (for example, cross-linking, physical adsorption, or entrapment) known to persons skilled in the art. The process for producing glyoxylic acid of the present invention can directly use a microorganism culture solution or cells without purification of the enzyme, and thus it is an advantageous production process for industrial production.

The reaction conditions depend on the type of the enzyme, the microorganism, or the treatment product, and the substrate concentration. However, preferably, the reaction is preformed in the presence of oxygen, the reaction temperature is 10°C to 70°C, and preferably 10°C to 50°C from the viewpoint of thermal stability, and the reaction pH is 4 to 12, and preferably 6 to 10 from the viewpoint of optimum pH and pH stability.

Although hydrogen peroxide is produced in the oxidation of glycolic acid to glyoxylic acid with oxidase, the hydrogen peroxide may deactivate oxygen and decompose glyoxylic acid into formic acid. However, the hydrogen peroxide produced by oxidase reaction can be decomposed and removed by adding catalase to the reaction system, thereby preventing deactivation of the enzyme and decomposition of glyoxylic acid.

Although the present invention will be described in detail below with reference to examples, the present invention is not limited to these examples.

### Example 1

First, 5 ml of an S medium (pH 7) containing 10 g/L of glycolic acid, 0.1 g/L of a yeast extract, 2 g/L of ammonium nitrate, 1 g/L of dipotassium hydrogen phosphate, 1 g/L of sodium dihydrogen phosphate, 0.2 g/L of magnesium sulfate heptahydrate, and 0.1 g/L of calcium chloride dihydrate was placed in a test tube and sterilized by high pressure steam. Then, 2 g of each soil sample collected in Japan was suspended in 10 ml of physiological saline, and 0.2 ml of the supernatant was added to the S medium, followed by enrichment culture at 28°C for 3 to 7 days. Then, 0.1 ml of the culture solution containing grown bacteria was coated on each S medium plate containing 2% of agar, and subjected to culture at 28°C for 3 to 7 days. The cells in each of the grown colonies were subjected to shaking culture on 5 ml of an S medium in a test tube at 28°C for 3 days. Then, the bacterial cells were collected by centrifugation, washed with physiological saline, and then suspended in 0.5 ml of a 100 mM phosphate buffer (pH 7). Then, 0.1 ml of the cell suspension was added to 0.1 ml of a 100 mM phosphate buffer containing 100 mM of glycolic acid, 1.34 mM of 4-AA, 2.18 mM of TOOS, and 4 U/ml of peroxidase, followed by shaking at 28°C for 2 hours. The cells which changed the reaction solution to a purple color after the shaking, i.e., produced hydrogen peroxide by reaction with glycolic acid were obtained as a strain having positive activity for glycolic acid oxidation.

The resultant strain having positive activity for glycolic acid oxidation was inoculated into 5 ml of an S medium and subjected to shaking culture at 28°C for 2 days to prepare a preculture solution. Then, 0.5 ml of the preculture solution was inoculated into 50 ml of an S medium in a 500-ml Sakaguchi flask and subjected to shaking culture at 28°C for 3 days. Furthermore, cells were collected from 20 ml of the resultant culture solution by centrifugation and washed with physiological saline. Then, the cells were suspended in 5 ml of a 100 mM phosphate buffer (pH 7) containing 100 mM of glycolic acid, and the resultant suspension was shaken at 28°C for 6 hours. After the reaction, the supernatant was analyzed by high-performance liquid chromatography to confirm the production of glyoxylic acid and determine the amount of glyoxylic acid produced. As a result, a strain having an activity to convert glycolic acid to glyoxylic acid was obtained. The strain was named Arthrobacter sp. KNK-GA1 strain and deposited in International Patent Organism Depositary, National Institute of Advanced Industrial Science and Technology (address: Tsukuba Central 6, 1-1-1 Higashi, Tsukuba, Ibaraki, Japan (postal code 305-8566), on March 7, 2002 (accession number: FERM BP-8375).

### Example 2

In a 500-ml Sakaguchi flask, the KNK-GA1 strain (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375) which was isolated from soil was inoculated into 50 ml of a medium (pH 7) containing 5 g/L of glycolic acid, 1 g/L of a yeast extract, 3.5 g/L of potassium dihydrogen phosphate, 6.5 g/L of diammonium hydrogen phosphate, 0.5 g/L of magnesium sulfate heptahydrate, 0.02 g/L of zinc sulfate heptahydrate, 0.03 g/L of ferrous sulfate heptahydrate, 0.002 g/L of copper sulfate pentahydrate, 0.1 g/L of calcium chloride dihydrate, and 0.3 g/L of sodium chloride. After shaking culture at 28°C for 2 days, cells were collected in a test tube from 10 ml of the resultant culture solution by centrifugation, and then suspended in 2 ml of a 100 mM phosphate buffer (pH 7). Then, 0.1 ml of a 1 M glycolic acid solution was added to 0.9 ml of the cell suspension, followed by shaking reaction at 28°C for 16 hours in a test tube. The resultant reaction solution was analyzed by high-performance liquid chromatography. As a result, 30 mM of glyoxylic acid was produced.

The high-performance liquid chromatographic analysis was performed using a BioRad Aminex HPX-87H (7.8 mm x 300 mm) column and a 5 mM H₂SO₄ aqueous solution as a solvent at a flow rate of 0.4 ml/min. Detection was performed by measuring absorbance at 230 mM.

### Example 3

First, cells were collected by centrifugation from 50 ml of the culture solution of the KHK-GA1 strain (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375) which was prepared by the method in Example 2. The cells were washed with a 0.1 M phosphate buffer (pH 7), and then suspended in 4 ml of a 0.1 M phosphate buffer (pH 7). Then the resultant cell suspension was crushed with a Mini-Bead Beater (produced by BIOSPEC Products, Inc.), and centrifuged to prepare a supernatant (cell-free extract). In a test tube, 0.2 ml of a 1 M glycolic acid solution and 0.1 ml of a 40,000 U/ml catalase solution were added to 1.7 ml of the cell-free extract, followed by shaking at 28°C for 4 hours. The high-performance liquid chromatographic analysis of the resultant reaction solution showed the production of 40 mM of glyoxylic acid.

### Example 4

First, 2 ml of the cell-free extract prepared in Example 3 was dialyzed with 4 L of a 0.1 M phosphate buffer. After the dialysis, 0.2 ml of a 1 M glycolic acid solution and 0.1 ml of a 40,000 U/ml catalase solution were added to 1.7 ml of the cell-free extract, and the resultant mixture was shaken in a test tube at 28°C for 4 hours. The high-performance liquid chromatographic analysis of the resultant reaction solution showed the production of 32 mM of glyoxylic acid.

### Example 5

First, 0.05 ml of a 0.1 M phosphate buffer (pH 7) containing 1.34 mM of 4-AA, 2.19 mM of TOOS, and 6 U/ml of POD was added to 0.1 ml of the cell-free extract produced after dialysis in Example 4. Furthermore, 0.05 ml of a 100 mM glycolic acid solution was added to the resultant mixture, followed by shaking in a test tube at 28°C for 2 minutes. As a result, the reaction solution turned to a dark purple color. As a control, 0.05 ml of a 0.1 M phosphate buffer was added instead of a 100 mM glycolic acid solution. In this case, the reaction solution did not show a change in color. It was thus confirmed that hydrogen peroxide was produced by oxidation of glycolic acid with the cell-free extract, and that an enzyme functioning as a catalyst for oxidation of glycolic acid was oxidase.

### Example 6

In a 500-ml Sakaguchi flask, the Arthrobacter sp. KNK-GA1 strain (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375) which was isolated from soil was inoculated into 100 ml of a medium (pH 7) containing 20 g/L of glucose, 1 g/L of a yeast extract, and 8 g/L of Nutrient broth. Then, the strain was cultured at 28°C for 1 day to prepare a preculture solution. In a 5 L minijar, the preculture solution was inoculated into 3 L of a medium (pH 7) containing 5 g/L of glycolic acid, 1 g/L of a yeast extract, 3.5 g/L of potassium dihydrogen phosphate, 6.5 g/L of diammonium hydrogen phosphate, 0.5 g/L of magnesium sulfate heptahydrate, 0.02 g/L of zinc sulfate heptahydrate, 0.03 g/L of ferrous sulfate heptahydrate, 0.002 g/L of copper sulfate pentahydrate, 0.1 g/L of calcium chloride dihydrate, 0.3 g/L of sodium chloride. Then, culture was performed at 0.5 vvm, 350 rpm, and pH 7.2 or less (controlled with an aqueous solution of sodium hydroxide) for 45 hours. During the culture, 15 g each of glycolic acid was added 7 hours, 21 hours, 27 hours, and 32 hours after the start of the culture.

Then, cells were collected from the resultant culture solution by centrifugation, and suspended in 1.2 L of a 0.05 M phosphate buffer (pH 7).

The resulting cell suspension was crushed by Dynomill and then centrifuged to obtain 1.1 L of a supernatant. Then, 62 ml of a 5% aqueous solution of protamine sulfate was added to the supernatant, and the mixture was stirred for 30 minutes and centrifuged to remove precipitates. As a result, 1.2 L of a supernatant was obtained.

Then, ammonium sulfate was gradually added to 1.2 L of the supernatant under stirring with a stirrer under ice cooling. When the degree of saturation of ammonium sulfate added was in the range of 25% to 40%, the precipitated protein was collected by centrifugation.

The protein precipitated with ammonium sulfate was dissolved in 320 ml of a 0.05 M phosphate buffer (pH 7), and dialyzed with 15 L of the same buffer. Then, the protein solution was charged in a DEAE-Toyopearl 650M (produced by TOSOH Corporation) column (diameter: 4 cm, height: 20 cm) previously equilibrated with a 0.05 M phosphate buffer (pH 7.2), and subjected to elution with a linear concentration gradient from 0 M to 0.2 M sodium chloride and at a flow rate of 90 ml/hr to obtain an active fraction. Then, a 0.05 M phosphate buffer containing 1.2 M of ammonium sulfate was added to the obtained fraction so that the ammonium sulfate concentration was 0.6 M. The resultant mixture was charged in a Phenyl-Toyopearl 650M (produced by TOSOH Corporation) column (diameter: 2.4 cm, height: 24 cm) previously equilibrated with a 0.05 M phosphate buffer (pH 7.2) containing 0.6 M ammonium sulfate, and subjected to elution with a linear concentration gradient from 0.6 M to 0 M ammonium sulfate and at a flow rate of 70 ml/hr to obtain an active fraction. The resultant fraction was then dialyzed with a 0.05 M phosphate buffer (pH 7). The solution after the dialysis was charged in a SuperQ-Toyopearl 650M (produced by TOSOH Corporation) column (diameter: 2.4 cm, height: 20 cm) previously equilibrated with a 0.05 M phosphate buffer (pH 7.2), and subjected to elution with a linear concentration gradient from 0 M to 0.3 M sodium chloride and at a flow rate of 50 ml/hr to obtain an active fraction. The active fraction was adjusted to an ammonium sulfate concentration of 0.4 M, and then charged in a Butyl-Toyopearl 650 M (produced by TOSOH Corporation) column (diameter: 1.4 cm, height: 10 cm) previously equilibrated with a 0.05 M phosphate buffer (pH 7.2) containing 0.4 M of ammonium sulfate, and subjected to elution with a linear concentration gradient from 0.4 M to 0 M ammonium sulfate and at a flow rate of 30 ml/hr to obtain an active fraction. The active fraction was adjusted to an ammonium sulfate concentration of 0.6 M, and then charged in a RESOURCE 15PHE (produced by Amersham Pharmacia Biotech Corporation) column (6 ml) previously equilibrated with a 0.05 M phosphate buffer (pH 7.2) containing 0.6 M of ammonium sulfate, and subjected to elution with a linear concentration gradient from 0.6 M to 0 M ammonium sulfate and at a flow rate of 2 ml/min to obtain an active fraction.

The resultant active fraction was subjected to SDS polyacrylamide gel electrophoresis analysis. As a result, a single band was formed at a position corresponding to a molecular weight of 60,000 Da.

### Example 7

The enzymatic properties of the enzyme produced in Example 6 were measured.

The enzyme activity was basically measured by reacting 1.0 ml of a reaction solution containing 10 mM of a substrate such as glycolic acid, 0.67 mM of 4-AA, 1.09 mM of TOOS, 2 U/ml of POD, and 0.1 ml of an enzyme solution in a 100 mM phosphate buffer or Tris buffer at 30°C for 100 seconds, and then measuring an increase in absorbance at a wavelength of 555 nm.

### (Substrate specificity)

As a result of reaction with each of the various alcohols and aldehydes shown in Table 2 used as a substrate, the enzyme of the present invention had the substrate specificity shown in Table 2.

**Table 2**

| Substrate specificity of enzyme of the present invention | |
|---|---|
| Substrate 10 mM | Relative activity (%) |
| Glycolic acid | 100 |
| D-lactic acid | 82 |
| L-lactic acid | 0 |
| DL-2-hydroxy-n-butyric acid | 42 |
| DL-2-hyrdoxyvaleric acid | 32 |
| DL-2-hydroxycaproic acid | 7 |
| DL-2-hydroxyisocaproic acid | 10 |
| L-2-hydroxyisocaproic acid | 0 |
| DL-glyceric acid | 54 |
| β-hydroxypropionic acid | 1 |
| DL-malic acid | 0 |
| Methanol | 0 |
| Ethanol | 0 |
| 1-propanol | 0 |
| 2-propanol | 0 |
| Ethylene glycol | 0 |
| 1,2-propanediol | 0 |
| Glycerin | 0 |

### (Thermal stability)

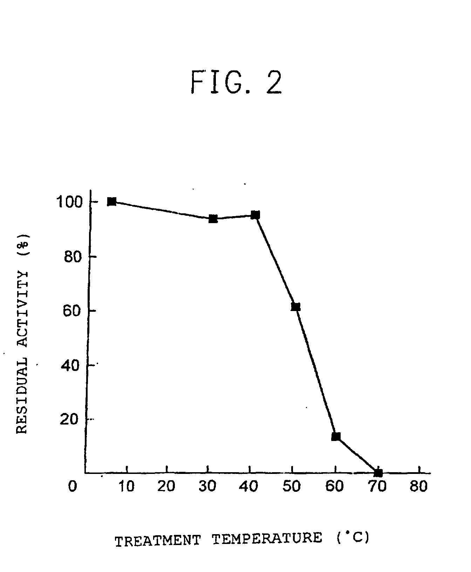

After treatment in a 0.05 M phosphate buffer (pH 7) at 30°C to 70°C for 20 minutes, the activity was measured using glycolic acid as a substrate. The results are shown in Fig.2 indicate that the residual activity after treatment at 30°C to 50°C was 90% or more of activity before the treatment.

### (Optimum reaction pH)

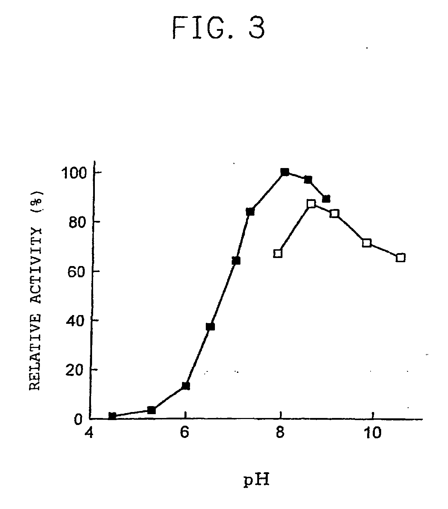

The activity was measured using glycolic acid as a substrate and a 1 M phosphate buffer and a 0.1 M Tris-hydrochloric acid buffer in the pH range of 5 to 10. The results are shown in Fig. 3. The optimum pH was 7 to 9.

### (pH stability)

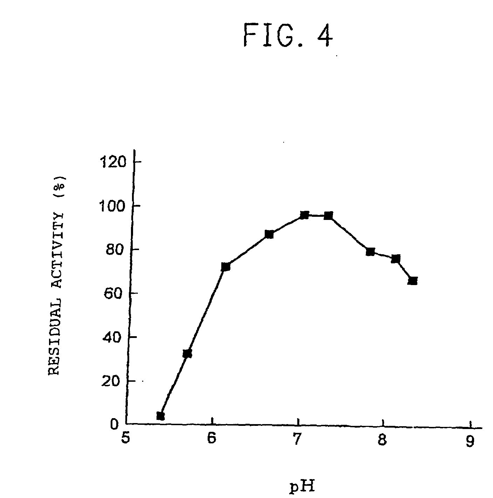

After storage in a 0.05 M phosphate buffer at pH 5.4 to 8.3 and at 5°C for 24 hours, the activity was measured using glycolic acid as a substrate, and compared with the activity before the storage. The results are shown in Fig. 4.

### (Molecular weight)

The purified enzyme of the present invention was subjected to 10% SDS-polyacrylamide gel electrophoresis analysis in the presence of 1% of 2-mercaptoethanol, and the molecular weight was estimated from relative mobility to a standard protein. As a result, the enzyme formed a single band at a position corresponding to a molecular weight of about 60,000 Da.

### Example 8

In a test tube, 1 ml of a 200 mM phosphate buffer (pH 7) containing 1 U of the purified enzyme produced in Example 6, 15 mg of glycolic acid, and 4000 U of catalase was subjected to shaking reaction at 20°C for 5 hours, and the reaction solution was analyzed by high-performance liquid chromatography. As a result, 3.5 mg of glyoxylic acid and 0.02 mg of formic acid were produced. However, oxalic acid was not detected.

The high-performance liquid chromatographic analysis was performed by using a BioRad Aminex HPX-87H (7.8 mm x 300 mm) column and a 5 mM H₂SO₄ aqueous solution as a solvent at a flow rate of 0.4 ml/min. Detection was performed by measuring absorbance at 230 mM.

### Example 9

In a test tube, 1 ml of a 200 mM phosphate buffer (pH 7) containing 1 U of the purified enzyme produced in Example 6, 15 mg of glycolic acid, and 0 U or 4000 U of catalase was subjected to shaking reaction at 20°C for 2 hours, and the reaction solution was analyzed by high-performance liquid chromatography. As a result, when 4000 U of catalase was added, 2.3 mg of glyoxylic acid and 0.02 mg of formic acid were produced. However, when catalase was not added, only 0.08 mg of glyoxylic acid was accumulated, and 1.6 mg of oxalic acid was produced.

### Comparative Example 1

The activity of glycolate oxidase (produced by Sigma Corporation) derived from spinach was examined for various 2-hydroxy acid compounds, and the results were compared with the results of the enzyme of the present invention produced in Example 7.

**Table 3**

| Comparison of substrate specificity between glycolate oxidase derived from spinach and enzyme of the present invention | | |
|---|---|---|
| Substrate 10 mM | Relative activity (%) | |
| | Enzyme of this invention | Spinach |
| Glycolic acid | 100 | 100 |
| D-lactic acid | 82 | 0 |
| L-lactic acid | 0 | 81 |
| DL-2-hydroxy-n-butyric acid | 42 | 71 |
| DL-2-hydroxyvaleric acid | 32 | 70 |
| DL-2-hydroxycaproic acid | 7 | 29 |
| DL-2-hydroxyisocaproic acid | 10 | 51 |
| L-2-hydroxyisocaproic acid | 0 | 68 |
| DL-glyceric acid | 54 | 76 |

### Industrial Applicability

The (D)-2-hydroxy-acid oxidase of the present invention has the ability of effectively converting glycolic acid to glyoxylic acid. The process for producing glyoxylic acid from glycolic acid using the enzyme or microorganisms capable of producing the enzyme is capable of producing glyoxylic acid under mild conditions without producing by-products. The (D)-2-hydroxy-acid oxidase of the present invention is the first enzyme found to selectively act on D-2-hydroxy acids.

## Claims

1. (D)-2-Hydroxy-acid oxidase having the physicochemical properties (1) and (2):
(1) action: acting on a 2-hydroxy acid to produce a corresponding 2-keto acid; and
(2) substrate specificity: exhibiting activity for glycolic acid and D-lactic acid, but no activity for L-lactic acid.

2. The (D)-2-hydroxy-acid oxidase according to claim 1, further having the physicochemical property (3):
(3) exhibiting no activity for L-2-hydroxyisocaproic acid, methanol, ethanol, 1-propanol, 2-propanol, ethylene glycol, 1,2-propanediol, and glycerin.

3. The (D)-2-hydroxy-acid oxidase according to claim 1 or 2, further having the physicochemical properties (4) to (6):
(4) molecular weight: about 60,000 Da in SDS polyacrylamide gel electrophoresis analysis;
(5) thermal stability: maintaining 90% or more of activity after treatment at pH 7.2 and 40°C for 20 minutes; and
(6) optimum reaction pH: 7 to 10.

4. The (D)-2-hydroxy-acid oxidase according to any one of claims 1 to 3, obtainable from a microorganism of the genus Arthrobacter.

5. The (D)-2-hydroxy-acid oxidase according to claim 4, wherein the microorganism of the genus Arthrobacter is Arthrobacter sp. KNK-GA1 (depository institution: International Patent Organism Depositary, National Institute of Advanced Industrial Science and Technology, address: Tsukuba Central 6, 1-1-1 Higashi, Tsukuba, Ibaraki, Japan (postal code 305-8566), deposit date: March 7, 2002, accession number: FERM BP-8375).

6. A process for producing glyoxylic acid comprising treating glycolic acid with any one of 2-hydroxy-acid oxidase, a culture solution of a microorganism capable of producing the enzyme, microorganism cells isolated from the culture solution, and a treatment product of the microorganism cells to convert the glycolic acid to glyoxylic acid, and collecting the glyoxylic acid.

7. The process for producing glyoxylic acid according to claim 6, wherein the reaction is preformed in the presence of catalase.

8. The process for producing glyoxylic acid according to claim 6 or 7, wherein the 2-hydroxy-acid oxidase is the (D)-2-hydroxy acid oxidase according to any one of claims 1 to 5.
